Europäisches Patentamt

European Patent Office

Office européen des brevets

(19)

(11) Publication number: **0 039 124**
**B1**

(12)  EUROPEAN PATENT SPECIFICATION

(45) Date of publication of patent specification: 26.09.84

(51) Int. Cl.³: **A 61 M 5/14, A 61 F 1/00**

(21) Application number: **81300951.1**

(22) Date of filing: **06.03.81**

(54)  **Implantable infusion apparatus.**

(30) . Priority: **07.03.80 US 127944**

(43) Date of publication of application:
**04.11.81 Bulletin 81/44**

(45) Publication of the grant of the patent:
**26.09.84 Bulletin 84/39**

(84) Designated Contracting States:
**BE CH DE FR GB IT LI NL SE**

(56) References cited:
**WO-A-81/00209**
**US-A-4 077 405**
**US-A-4 193 397**

(73) Proprietor: **INFUSAID CORPORATION**
**1400 Providence Highway**
**Norwood, MA 02062 (US)**

(72) Inventor: **Tucker, Elton M.**
**8 Oxbow Road**
**Medfield Massachusetts 02052 (US)**

(74) Representative: **Orchard, Oliver John**
**JOHN ORCHARD & CO. Staple Inn Buildings**
**North High Holborn**
**London WC1V 7PZ (GB)**

## Description

This invention relates to apparatus for controlledly dispensing infusate to a living body over the long term. It relates more particularly to such apparatus which is implantable in the living body.

Relatively recently, infusion apparatus has been developed which can be implanted in the body to remain there for a prolonged period. The apparatus can be refilled with infusate without having to remove the apparatus. Such refilling is achieved simply to injecting additional infusate through a penetrable septum in the apparatus, which septum is located directly under the patient's skin. In some cases, the act of refilling the apparatus with infusate also recharges its power source so that the device can operate uninterruptedly to dispense infusate such as insulin or heparin at a very small flow rate for a prolonged period. An example of infusion apparatus of this general type is disclosed in U.S. patent 3,731,681.

It has also been proposed to control the infusate flow from such apparatus by means of a valve controlled by a programmable controller so that the dosage rate can be varied to suit the particular needs of the patient. For example, in the case of insulin, ideally the patient should receive a small continuous or basal dose to satisfy a particular steady state requirement that depends upon the weight of the patient and the average amount of sugar in his blood. Then immediately after a meal when blood sugar level rises dramatically, the infusion apparatus should dispense a much larger dose (prolonged bolus) of insulin to offset the increased sugar level caused by the ingestion of that meal. One such programmable infusion apparatus, described in U.S. Patent 4,077,405, varies the valve control pulses in frequency and/or duration to control the duty cycle of an electrically operated valve to provide the requisite basal and bolus doses at the required times.

One problem with such prior apparatus which rely on electrically operated controllers to regulate both the basal and bolus infusion rates to the patient is that they consume a relatively large amount of energy so that the energy source which is a battery must be recharged or replaced relatively often.

Prior controllable infusion apparatus is disadvantaged also in that it is possible for the patient to inadvertently or intentionally control the apparatus so as to administer an overdose of infusate. This not only exhausts the supply of infusate, but also can result in injury to the patient. An insulin overdose, for example, can bring on hypoglycemic shock resulting in death to the patient.

The arrangements of both the present invention and of US—A—4 077 405 employ a fluid accumulator and two flow paths between a reservoir and a fluid outlet, with a control valve in one of the paths. However, the arrangement of the present invention is distinguished by the fact that the accumulator is connected to the second flow path between the reservoir and the control valve, and the fact that a flow restricter is included in the second flow path between the reservoir and the accumulator. By means of this arrangement the risk of a patient inadvertently obtaining an overdose is minimised, as will be explained in more detail below.

More generally, however, it would be desirable to provide implantable infusion apparatus which is small and compact so as to occupy a minimum amount of space in the body, yet which is able to supply the patient's basal requirements over the long term plus periodic bolus doses as needed in a controlled fashion while still avoiding the need for surgically removing the apparatus in order to refill it with infusate or to replace or recharge its power supply.

It is an object of the present invention, therefore, to provide implantable infusion apparatus which can dispense infusate to a selected site in the body at a small flow rate continuously for a prolonged period.

Another object of the invention is to provide infusion apparatus of this type which can provide both the basal and bolus infusate needs of a patient for a prolonged period without having to be surgically removed from the patient.

Another object of the invention is to provide such apparatus whose bolus infusion is patient-actuated, yet which avoids the possibility of a patient-administered infusate overdose.

Another object is to provide such apparatus whose bolus infusate flow can be controlled to suit patient's needs.

Yet another object is to provide an infusion device which permits great flexibility in the selection of the bolus dosage to the patient.

Other objects will, in part, be obvious and will, in part, appear hereinafter.

The invention accordingly comprises the features of construction, the combination of elements and the arrangement of parts which will be exemplified in the following detailed description, and the scope of the invention will be indicated in the claims.

Infusion apparatus of the type with which we are concerned here has particular application as a so-called artificial pancreas to dispense insulin to a diabetic patient to counteract excessive glucose present in the patient's bloodstream. Accordingly we will describe the invention in that context. It should be understood, however, that the apparatus can be used to dispense a variety of other infusates, heparin being one example, into a human or animal body for various purposes.

The average diabetic should receive a basal dosage of insulin continuously in response to changes in the level of glucose in the bloodstream. In addition, he should receive larger, short-term, so-called bolus doses of insulin to offset much higher, short-term glucose levels in the bloodstream which may be present particularly after meals. Moreover, the bolus doses should be introduced and take effect as soon as possible and should terminate as the glucose level is returned to its basal state. The present apparatus accomplishes these objectives with a small compact package which occupies a minimum amount of space in the body and which requires a minimum amount of energy so that it can remain in the patient's body for a relatively long time.

The apparatus employs a single infusate reservoir from which both the basal and bolus infusate doses are dispensed to the infusion site in the patient's body along parallel paths having different fluid flow characteristics. Preferably, the infusate reservoir is of the type comprising a variable volume infusate chamber, e.g. a bellows capsule, which is collapsed by the pressure exerted by a confined fluid on the chamber walls to expel the infusate from the chamber. Most preferably, the fluid is a two-phase fluid which vaporizes at physiological temperatures so as to exert the requisite pressure on the chamber walls such as is disclosed in U.S. patent 3,731,681. However, other fluids or gases or even a spring may be used to collapse the chamber to expel the infusate. In that event, a suitable pressure regulator should be provided at the chamber outlet to maintain constant flow despite changes in the patient's body temperature or in the ambient pressure.

The reservoir outlet is connected to a small mixing chamber by a first flow path having a first flow restriction characteristic which satisfies the patient's long-term basal infusate requirement. Also, leading from the reservoir to the chamber is a second flow path parallel to the first and having a lesser restriction characteristic. A normally closed valve is connected in the second flow path. This valve is opened at the appropriate times by electrical signals from a controller mounted in the apparatus housing. When the valve is opened, the bolus flow to the chamber supplements the basal flow thereto so that a combined or integrated dose is available to the patient. An outlet tube from the chamber conducts the infusate to a catheter located at the infusion site in the patient's body.

Preferably, the controller is programmable to vary the frequency and/or pulse duration of its output signal so as to vary the valve duty cycle to suit the bolus needs of the particular patient. Preferably also, the output signals from the controller are initiated by the patient himself by his actuating a switch mounted in the apparatus housing and positioned directly below the skin. Upon such actuation, the controller issues control signals to the valve causing it to open and close in a controlled fashion so that the requisite amount of infusate flows from the infusate reservoir through the second flow path to the mixing chamber where it mixes with the basal dose, the combined dose then flowing to the infusion site in the patient's body. For example, at mealtime, the patient would activate the controller. The controller would then automatically open and close the valve so that the infusion apparatus would dispense an integrated infusate dose to the patient to offset the increased glucose level in his bloodstream caused by the ingestion of that meal. That dose would be tailored to that patient's particular needs, and would cease after some predetermined time or could be terminated by the patient.

The present apparatus also limits the bolus dose volume which the apparatus can dispense within a selected time period to prevent possible injury to the patient. For example, a person may not remember that he has actuated the controller switch and received a bolus dose after a given meal and may actuate it again two or more times within a short time interval. To avoid this potential problem, the present apparatus includes an auxiliary infusate reservoir or accumulator in the second fluid path upstream from its valve. This reservoir may be similar to the main reservoir but it is quite small. Also a flow restrictor is located in the second path between the main reservoir and the auxiliary reservoir to limit infusate flow from the reservoir to the auxiliary reservoir so that the latter cannot be refilled more than once in a given time period, e.g. four to seven hours. Therefore, each time the patient actuates the controller switch to obtain a bolus dose of infusate, the maximum amount of infusate that he can obtain within that time period is the contents of the auxiliary reservoir, which contents will, for example, comprise one bolus dose or less. Thus, if the patient actuates the switch once after a given meal and, due to forgetfulness, actuates it again somewhat later, he will not receive any appreciable additional infusate above and beyond the usual basal dose because the auxiliary reservoir will not have had time to refill from the main reservoir.

With the present system, then, the infusate concentration, the reservoir and mixing chamber volumes and the flow rates to and from the mixing chamber are selected to provide an integrated dosage profile from the apparatus which is tailored to the patient's requirements. Thus the apparatus can provide both the long-term continuous basal infusate requirement of the patient and his intermittent, larger bolus needs for a prolonged period between apparatus refills. Yet the apparatus is relatively small and compact so that it causes a minimum amount of discomfort to the patient.

Another advantage of the present infusion apparatus stems from the fact that all of the energy required to pump infusate to the infusion site is provided by pressurizable fluids or by a fluid power cell, which energy source is automatically recharged each time the reservoir is refilled with infusate. Thus electrical energy is required only periodically to operate the controller and actuate the valve. Therefore, the apparatus can operate on a continuous basis for a long period of time (e.g. several years) before its battery requires replacement or recharging. As a result of the aforesaid advantages, the present implantable infusion apparatus should find wide application as a dispenser for insulin, hormones, drugs and other such beneficial fluids.

For a fuller understanding of the nature and objects of the invention, reference should be had to the following detailed description, taken in connection with the accompanying drawing, in which:

FIG. 1 is a schematic diagram of implantable infusion apparatus embodying the principles of this invention; and

FIGS. 2A to 2C are graphical diagrams of the valve function and flow rates of the FIG. 1 apparatus.

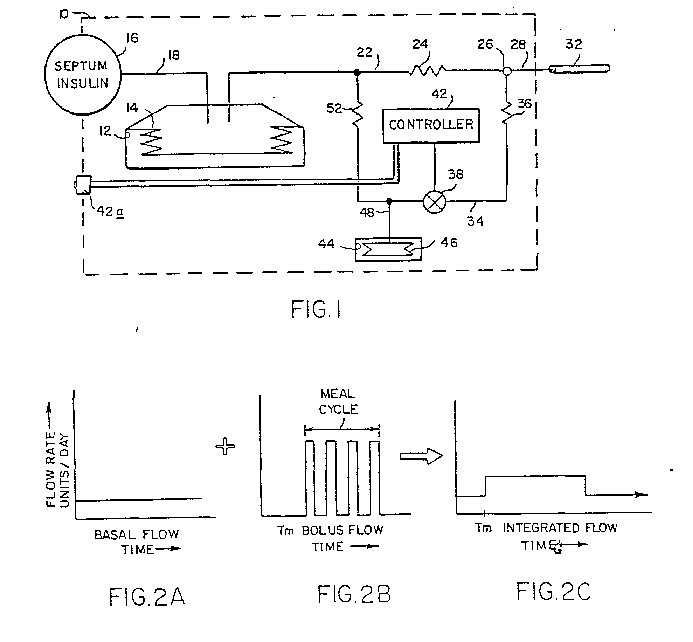

Referring to FIG. 1 of the drawing, the various apparatus components are enclosed within a housing shown in dotted lines at 10. Certain of the components are similar to the ones described in the specification of U.K. Patent Application Serial No. 2,028,275A published 5th March, 1980. Accordingly, they will not be described in detail here. Suffice it to say that the housing 10 interior is subdivided into a number of fluid-tight chambers of compartments. Mounted within a relatively large chamber 12 inside the housing is a bellows capsule 14. The capsule divides chamber 12 into two volumes, one being inside the capsule and the other being outside the capsule but within chamber 12. Furthermore, these volumes change in a reciprocal manner as the capsule is extended and compressed.

A penetrable self-sealing septum 16 is mounted in the housing wall so that the bellows capsule 14 can be filled with infusate by injection through septum 16 into a passage 18 leading to the interior of the bellows capsule. The space inside chamber 12 but outside the bellows capsule is filled with a pressurizable fluid. The fluid may be of the two-phase variety as described in the aforesaid patents or a simple gas. In either event when the bellows capsule is filled with infusate, it expends, thereby compressing the gas in the chamber 12 so that the gas exerts pressure on the capsule and tends to expel infusate from the capsule via a first flow path 22. The pressure exerted on the capsule should exceed the patient's infusion site pressure to establish fluid flow. A suitable pressure regulator (not shown) may be provided at the capsule outlet to maintain constant flow despite changes in the ambient pressure or temperature. Also, an appropriate filter (not shown) is provided at the capsule outlet. The flow path 22 has a selected flow restriction indicated at 24 which permits infusate flow to a mixing chamber 26 at a very slow rate, e.g. on the order of 0.6 ml/day, the specific rate depending upon the particular patient's basal insulin requirement. From chamber 26, the infusate proceeds through tubing 28 to a catheter 32 and into the patient's physiological system.

Infusate from the bellows capsule 14 also flows to chamber 26 by way of a second flow path 34 which is parallel to path 22. Path 34 also has a selected flow restriction indicated at 36 which permits a faster infusate flow to chamber 26 to satisfy the patient's bolus requirement. Fluid flow along path 34 is normally blocked by a valve 38 whose operation is controlled by an electrical controller 42 activated by a switch button 42a located just under the skin. The valve may be a solenoid valve, a piezoelectric valve or other valve which responds to electrical signals. The controller is also of a conventional variety, a suitable one being described in U.S. Patent 4,077,405.

A second chamber 44 is formed in housing 10 which contains a second bellows capsule 46. Capsule 46 divides chamber 44 into two volumes, one being inside the capsule and the other being outside the capsule but within chamber 44. Fluid communication between the interior of bellows 46 and the flow path 34 upstream of valve 38 is established by a flow path 48. Also a flow restriction 52 is included in the flow path 34 between capsule 14 and capsule 46 to limit the flow rate to the latter capsule. The space inside chamber 44 but outside bellows capsule 46 is also filled with a pressurizable fluid which exerts less pressure on capsule 46 than is exerted on capsule 14. Yet that pressure still exceeds the infusion site pressure in the body. Furthermore, capsule 46 is arranged so that the amount of fluid that can be pumped from capsule 46 during a given stroke of that bellows is a small percentage of that delivered by a single stroke of capsule 14.

Thus infusate from capsule 14 is delivered via path 22 directly to chamber 26 and thence to catheter 32. It also flows via flow paths 34 and 48 to capsule 46, thereby expanding that capsule and compressing the fluid in chamber 44. Consequently, the infusate in capsule 46 is maintained under pressure so that when valve 38 is opened, infusate is expelled along path 34 to the mixing chamber 26. In chamber 26, the bolus flow mixes with and supplements the basal infusate flow from path 22 and the combined dosage is delivered to catheter 32.

Refer now to FIGS. 2A to 2C which depict graphically the basal, bolus and integrated unit flow rates of the apparatus in operation. For purposes of this description, we will assume that the capsule 14 has been completely filled and charged by injection of infusate in the basal concentration through septum 16. The filling and charging of the main reservoir also results in the filling and extension of the capsule 46 in opposition to the gas pressure or a spring bias thereon until the acumulator is fully extended. In which position it may hold, for example, one bolus infusion dose for a particular patient. The valve 38 being normally closed prevents infusate flow from the accumulator to the mixing chamber 26. Accordingly, as seen from FIG. 2A, the patient receives a continuous infusate dose at the predetermined basal rate.

The patient can initiate the bolus dose by actuating switch 42a. The switch activates the controller 42 which emits a train of electrical pulses to valve 38 causing that valve to open a predetermined number of times each opening being for a selected duration as shown in FIG. 2B. Such openings of the valve permits the capsule 46 to expel its contents as pronounced infusate slugs through the restrictor 36 to the mixing chamber 26. In that chamber, the bolus infusate flow mixes with and supplements the basal flow still arriving directly from capsule 14, with the combined or integrated infusate dosage to the patient being reflected in FIG. 2C.

Referring again to FIG. 1, the present apparatus protects the patient from inadvertent bolus infusate overdoses in the event that the valve 38 is opened repeatedly or fails in the open position. More particularly, the maximum volume of bolus infusate that is available for delivery to the patient is the contents of capsule 46. This is because the flow restrictor 52 between capsule 14 and capsule 46 is such that the capsule 46 is refilled at a slow rate, e.g. over 4 to 5 hours. Accordingly, even if the valve 38 remains open for a long time, several hours, the patient still receives only a small portion of the bolus dose of infusate.

By the same token, if the patient actuated the controller at mealtime and received a bolus dose to offset the increased blood sugar level caused by that meal and then inadvertently actuates it again a short time thereafter, he will receive only a very small amount of infusate because the capsule 46 will not have been replenished with a significant amount of infusate from 14 during that time. Thus, the provision in the present apparatus of an accumulator located between an upstream low flow rate restrictor leading from the main reservoir and a downstream valve constitutes a significant safety feature in apparatus such as this.

Finally, since the capsules 14 and 46 are maintained under pressure, no electrical energy is required to deliver infusate to the catheter 32. Rather, electrical energy is only required to operate the valve 38 when bolus doses are required. Consequently, the duty cycle of the valve is short and its power requirements low as compared to prior devices which are pulsed more or less continuously. Therefore, the battery used to power controller 42 and valve 38 should last for a long time before requiring recharging or replacement.

It will thus be seen that the objects set forth above, among those made apparent from the preceding description, are efficiently attained, and, since certain changes may be made in the above construction without departing from the scope of the invention, it is intended that all matter contained in the above description or shown in the accompanying drawing be interpreted as illustrative and not in a limiting sense.

It will be understood that the pressurizable fluid acting upon the bellows 14 in the chamber 12 and that acting upon the bellows capsule 46 in the chamber 44 can each be considered to constitute a fluid power cell.

It is also to be understood that the following claims are intended to cover all of the generic and specific features of the invention herein described.

It will also be appreciated that the controller 42 may be programmable to vary the signals applied to the valve 38 in ways other than that described.

## Claims

1. Implantable infusion apparatus including an infusate reservoir (14), an infusate outlet (28) from the apparatus, a first flow path (22) connected between the reservoir (14) and the outlet (28), a second flow path (34) connected between the reservoir (14) and the outlet (28), a normally closed valve (38) in the second flow path, an infusate accumulator (46) which has a smaller volume than the reservoir (14) and is in fluid communication with the second flow path (34), first pressure means for exerting pressure on the reservoir (14) to expel infusate under pressure to the outlet (28), second pressure means for exerting pressure on the accumulator (40), means (42) for opening the valve (38) at selected time intervals, and means (32) for conducting the infusate from the outlet (28) to an infusion site, characterised in that the infusate accumulator (46) is connected to the second flow path (34) between the valve (38) and the reservoir (14) and in that there is a flow restrictor (52) in the second flow path (34) restricting flow between the reservoir (14) and the accumulator (46).

2. Apparatus as claimed in claim 1, characterised in that the first and second flow paths (22, 34) have different fluid flow restriction characteristics.

3. Apparatus as claimed in claim 2, characterised in that the second flow path (34) is less restrictive than the first flow path (22).

4. Apparatus as claimed in any one of the preceding claims, characterised in that the first pressure means is constituted by a fluid power cell.

5. Apparatus as claimed in any one of the preceding claims, characterised in that the said second pressure means exerts less pressure than is exerted by the first pressure means.

6. Apparatus as claimed in any one of the preceding claims, characterised in that the valve (38) opens in response to electrical signals, and in that there is provided an electrical controller (42) for applying electrical signals to the valve (38).

7. Apparatus as claimed in claim 6, characterised in that the controller (42) is programmable to vary the signals applied to the valve (38).

8. Apparatus as claimed in claim 6 or claim 7, characterised in that it includes means (42a) arranged to be actuatable extracorporally for activating the controller (42).

9. Apparatus as claimed in any one of the preceding claims, characterised in that it includes means (16) whereby the infusate reservoir can be refilled with infusate by subcutaneous injection.

## Revendications

1. Appareil de perfusion implantable, comportant un réservoir (14) de perfusat, une sortie (28) empruntée par le perfusat pour quitter l'appareil, un premier trajet d'écoulement (22) intercalé entre le réservoir (14) et la sortie (28), un second trajet d'écoulement (34) intercalé entre le réservoir (14) et la sortie (28), une valve (38) normalement fermée incorporée dans le second trajet d'écoulement, un accumulateur (46) de perfusat qui présente un volume plus petit que celui du réservoir (14) et est en communication par fluide avec le second trajet d'écoulement (34), un premier moyen exerçant une pression sur le réservoir (14) afin d'exprimer du perfusat sous pression vers la sortie (28), un second moyen exerçant une pression sur l'accumulateur (46), un dispositif (42) pour ouvrir la valve (38) à intervalles de temps choisis, ainsi qu'un moyen (32) pour acheminer le perfusat de la sortie (28) jusqu'à un site de perfusion, caractérisé par le fait que l'accumulateur (46) de perfusat est raccordé au second trajet d'écoulement (34) entre la valve (38) et le réservoir (14), et par le fait que le second trajet d'écoulement (34) renferme un limiteur de débit (52) qui réduit le débit entre le réservoir (14) et l'accumulateur (46).

2. Appareil selon la revendication 1, caractérisé par le fait que les premier et second trajets d'écoulement (22, 34) présentent des caractéristiques différentes de limitation du débit.

3. Appareil selon la revendication 2, caractérisé par le fait que le second trajet d'écoulement (34) exerce une limitation moindre que le premier trajet d'écoulement (22).

4. Appareil selon l'une quelconque des revendications précédentes, caractérisé par le fait que le premier moyen exerçant une pression consiste en une cellule développant une puissance à partir d'un fluide.

5. Appareil selon l'une quelconque des revendications précédentes, caractérisé par le fait que ledit second moyen de pression exerce une pression moindre que celle exercée par le premier moyen de pression.

6. Appareil selon l'une quelconque des revendications précédentes, caractérisé par le fait que la valve (38) s'ouvre en réaction à des signaux électriques, et par le fait qu'il est prévu un dispositif électrique de commande (42) pour délivrer des signaux électriques à cette valve (38).

7. Appareil selon la revendication 6, caractérisé par le fait que le dispositif de commande (42) peut être programmé pour faire varier les signaux appliqués à la valve (38).

8. Appareil selon la revendication 6 ou 7, caractérisé par le fait qu'il comporte un moyen (42a) disposé pour être actionnable à l'extérieur du corps en vue de mettre le dispositif de commande (42) en action.

9. Appareil selon l'une quelconque des revendications précédentes, caractérisé par le fait qu'il comporte un moyen (16) par l'intermédiaire duquel le réservoir de perfusat peut être rempli de perfusat par une injection sous-cutanée.

## Patentansprüche

1. Implantierbares Infusionsgerät mit einer Infusat-Vorratskammer (14), einem Infusat-Auslaß (28) aus dem Gerät, einer ersten Verbindungsleitung (22) zwischen der Vorratskammer (14) und dem Auslaß (28), einer zweiten Verbindungsleitung (34) zwischen der Vorratskammer (14) und dem Auslaß (28), einem normalerweise geschlossenen Ventil (38) in der zweiten Verbindungsleitung, einem Infusat-Zwischenspeicher (46) mit einem kleineren Volumen als die Vorratskammer (14), welcher Zwischenspeicher mit der zweiten Verbindungsleitung (34) verbunden ist, einer ersten druckerzeugenden Einrichtung zur Ausübung eines Drucks auf die Vorratskammer (14), um Infusat zu dem Auslaß (28) zu drücken, einer zweiten druckerzeugenden Einrichtung zur Ausübung eines Drucks auf den Zwischenspeicher (46), einer Einrichtung (42) zum Öffnen des Ventils (38) während ausgewählten Zeitintervallen, sowie mit einer Einrichtung (32) zum Weiterleiten des Infusats von dem Auslaß (28) zu einer Infusionsstelle, dadurch gekennzeichnet, daß der Infusat-Zwischenspeicher (46) mit der zweiten Verbindungsleitung (34) zwischen dem Ventil (38) und der Vorratskammer (14) verbunden ist, und daß ein Durchflußbegrenzer (52) in der zweiten Vergindungsleitung (34) vorgesehen ist, der den Durchfluß zwischen der Vorratskammer (14) und dem Zwischenspeicher (46) begrenzt.

2. Infusionsgerät nach Anspruch 1, dadurch gekennzeichnet, daß die beiden Verbindungsleitungen (22, 34) eine unterschiedliche Durchflußbegrenzungs-Charakteristik aufweisen.

3. Infusionsgerät nach Anspruch 2, dadurch gekennzeichnet, daß die Durchflußbegrenzung in der zweiten Verbindungsleitung (34) geringer als in der ersten Verbindungsleitung (22) ist.

4. Infusionsgerät nach einem der vorhergehenden Ansprüche, dadurch gekennziechnet, daß die erste druckerzeugende Einrichtung ein komprimiertes Druckmedium enthält.

5. Infusionsgerät nach einem der vorhergehenden Ansprüche, dadurch gekennzeichnet, daß die zweite druckerzeugende Einrichtung einen geringeren Druck als die erste druckerzeugende Einrichtung ausübt.

6. Infusionsgerät nach einem der vorhergehenden Ansprüche, dadurch gekennzeichnet, daß das Ventil (38) in Abhängigkeit von elektrischen Signalen öffnet, und daß eine elektrische Steuereinrichtung (42) zur Zufuhr von elektrischen Signalen zu dem Ventil (38) vorgesehen ist.

7. Infusionsgerät nach Anspruch 6, dadurch gekennzeichnet, daß die Steuereinrichtung (42) programmierbar ist, um die dem Ventil (38) zuführbaren Signale zu bestimmen.

8. Infusionsgerät nach Anspruch 6 oder 7, dadurch gekennzeichnet, daß zur Aktivierung der Steuereinrichtung (42) eine Einrichtung (42a) vorgesehen ist, die von der Außenseite des Körpers betätigbar ist, in den das Infusionsgerät implantiert ist.

9. Infusionsgerät nach einem der vorhergehenden Ansprüche, dadurch gekennzeichnet, daß es eine Einrichtung (16) enthält, durch die die Infusat-Vorratskammer durch subkutane Injektion nachfüllbar ist.

FIG.1

FIG.2A

FIG.2B

FIG.2C